# EUROPÄISCHE PATENTSCHRIFT

(11) **EP 2 252 567 B1**
(45) Veröffentlichungstag und Bekanntmachung des Hinweises auf die Patenterteilung: **07.08.2013**
(21) Anmeldenummer: 09717976.6
(22) Anmeldetag: 20.02.2009
(51) Int. Cl.: C07C 2/78, C07C 11/24

(54) **VERFAHREN UND VORRICHTUNG ZUR THERMISCHEN PARTIELLEN OXIDATION VON KOHLENWASSERSTOFFEN**
METHOD AND DEVICE FOR THERMAL PARTIAL OXIDATION OF HYDROCARBONS
PROCEDE ET DISPOSITIF D'OXYDATION THERMIQUE PARTIELLE D'HYDROCARBURES

(30) Priorität: 05.03.2008 EP 08152302
(43) Veröffentlichungstag der Anmeldung: 24.11.2010
(73) Patentinhaber: BASF SE, 67056 Ludwigshafen (DE)
(72) Erfinder: GROßSCHMIDT, Dirk, 68159 Mannheim (DE); VICARI, Maximilian, 67117 Limburgerhof (DE); WEICHERT, Christian, 67098 Bad Dürkheim (DE); ZAPF, Hans, 67112 Mutterstadt (DE); JOA, Andreas, 67435 Neustadt (DE); AGLAVE, Ravindra, 68165 Mannheim (DE); DENECKE, Jens, 67346 Speyer (DE)
(86) Internationale Anmeldenummer: PCT/EP2009/052052
(87) Internationale Veröffentlichungsnummer: WO 2009/109473

(56) Entgegenhaltungen:
- DE-A1- 4 422 815
- DE-A1-102005 018 981
- GB-A- 818 395
- GB-A- 824 328
- GB-A- 958 061
- US-A- 2 179 378
- US-A- 2 813 138
- US-A- 3 081 818

## Beschreibung

Die vorliegende Erfindung betrifft ein verbessertes Verfahren zur partiellen Oxidation von Kohlenwasserstoffen in einem Reaktor, bei welchem man dem Reaktor einen den Kohlenwasserstoff enthaltenden Strom sowie einen den Sauerstoff enthaltenden Strom zuleitet sowie eine Vorrichtung zur Durchführung des erfindungsgemäßen Verfahrens.

Hochtemperaturreaktionen zur partiellen Oxidation von Kohlenwasserstoffen werden üblicherweise in einem Reaktorsystemen enthalten Mischeinheit, Brenner und Quench durchgeführt.

Als Beispiel für so eine Partialoxidation im Hochtemperaturbereich ist die Herstellung von Acetylen und Synthesegas durch partielle Oxidation von Kohlenwasserstoffen zu nennen. Diese wird beispielsweise in DE 875198, DE 1051845, DE1057094 und DE 4422815 beschrieben.

Hierin werden die für das BASF-Sachsse-Barthotomé-Acetylenverfahren üblicherweise eingesetzten Mischer/Brenner/Feuerraum/Quench-Kombinationen - im folgenden, wenn auf die Kombination Bezug genommen wird, vereinfacht als "Reaktor" bezeichnet - erläutert.

Die Ausgangsstoffe Erdgas und Sauerstoff werden getrennt aufgeheizt, üblicherweise möglichst bis auf 700°C. In einer Mischzone werden die Reaktanden intensiv vermischt und nach Durchströmen eines Brennerblocks zur Reaktion gebracht. Der Brennerblock besteht in diesen Fällen aus einer bestimmten Anzahl aus parallelen Kanälen, in denen die Strömungsgeschwindigkeit der zündfähigen Sauerstoff/Erdgas-Mischung höher ist als die Flammengeschwindigkeit (Reaktionsgeschwindigkeit, Umsetzungsgeschwindigkeit), um ein Durchschlagen der Flamme in den Mischraum zu verhindern. Der metallische Brennerblock wird gekühlt, um den thermischen Belastungen standzuhalten. Je nach Aufenthaltszeit im Mischraum entsteht die Gefahr der Vor- und Rückzündung aufgrund der begrenzten thermischen Stabilität der Mischungen. Hier wird der Begriff der Zündverzugszeit, bzw. Induktionszeit gebraucht als die Zeitspanne, in der eine zündfähige Mischung keine nennenswerte intrinsische thermische Veränderung durchläuft. Die Induktionszeit ist abhängig von der Art der eingesetzten Kohlenwasserstoffe, dem Mischungszustand, von Druck und Temperatur. Sie bestimmt die maximale Aufenthaltszeit der Reaktanden im Mischraum. Reaktanden wie Wasserstoff, Flüssiggas oder Leichtbenzin, deren Einsatz aufgrund von Ausbeute- und /oder Kapazitätssteigerungen im Syntheseprozess besonders wünschenswert ist, zeichnen sich durch eine vergleichsweise hohe Reaktivität und damit geringe Induktionszeit aus.

Die im heutigen Produktionsmaßstab eingesetzten Acetylenbrenner zeichnen sich durch ihre zylinderförmige Geometrie des Feuerraums (Reaktors) aus. Der Brennerblock weist vorzugsweise hexagonal angeordnete Durchführungsbohrungen auf. In einer Ausführungsform sind z.B. 127 Bohrungen ä 27 mm Innendurchmesser hexagonal auf einem kreisförmigen Grundquerschnitt mit Durchmesser von ca. 500 mm angeordnet. In der Regel liegen die eingesetzten Kanaldurchmesser bei etwa 19 bis 27 mm Durchmesser. Der anschließende Feuerraum, in der die Flamme der acetylenbildenden partiellen Oxidationsreaktion stabilisiert wird, ist ebenfalls von zylindrischem Querschnitt und entspricht im Erscheinungsbild dem eines kurzen Rohres (von z.B. 533 mm Durchmesser und 400 mm Länge). Der gesamte Brenner aus Brennerblock und Feuerraum wird in einen Quenchbehälter größeren Querschnitts über einen Flansch von oben eingehängt. Auf Höhe der Austrittsebene aus dem Feuerraum sind außerhalb von dessen Umfang Quenchdüsen auf einem oder mehreren Quenchverteilerringen installiert, die das Quenchmedium, z.B. Wasser oder Öl, mit oder ohne Zuhilfenahme eines Zerstäubungsmedium zerstäuben und näherungsweise senkrecht zur Hauptströmungsrichtung der den Feuerraum verlassenden Reaktionsgase eindüsen. Dieser direkte Quench hat die Aufgabe, die reagierende Strömung extrem schnell abzukühlen, so dass Folgereaktionen, d.h. insbesondere der Abbau von gebildetem Acetylen, eingefroren werden. Die Reichweite und Verteilung der Quenchstrahlen ist dabei idealerweise so bemessen, dass eine möglichst homogene Temperaturverteilung in möglichst kurzer Zeit erreicht wird.

Die im aktuellen Produktionsmaßstab eingesetzten Acetylenbrenner zeichnen sich durch eine zylinderförmige Geometrie des Feuerraums aus. Die Einsatzstoffe werden über einen Diffusor vorgemischt und unter Vermeidung von Rückvermischung dem Brennerblock über hexagonal angeordnete Durchführungsbohrungen zugeführt. Bei den bekannten Verfahren erfolgt die Vormischung der Einsatzstoffe im Mischdiffusor in einem relativ großen Volumen und unter hohen Vorwärmtemperaturen. Je nach Kapazität des Brenners zeichnet sich der Mischdiffusor durch eine Bauform aus, in dem die Aufenthaltszeit der Reaktanden und deren Induktionszeiten in der gleichen Größenordnung liegen. Aufgrund eines erhöhten Anteils reaktiver Einsatzstoffkomponenten, katalytisch wirkender Partikel und Oberflächen, z.B. Koks, Rost, etc. kann es passieren, dass die Induktionszeiten für die Zündung des Gemisches überschritten werden. Diese Vorzündungen führen zu Betriebsunterbrechungen und damit zu einer Herabsetzung der Effektivität und Wirtschaftlichkeit des Prozesses.

In DE 10 2005 018 981 A1 oder US 2 179 378 sind Vorrichtungen beschrieben, welche die Einbringung der Reaktandenströme auf kleinem Raum in die unmittelbare Nähe der Brennerblockbohrungen verlegen. Die Brennerblockbohrungen dienen damit als Mischrohre, in denen das Reaktandengemisch ausgebildet wird. Durch eine geeignete Gestaltung der Einmischgeometrie werden hohe Geschwindigkeiten geschaffen, welche ein Rückzünden der Flamme in die Brennerblockbohrungen verhindern und gleichzeitig eine zügige Gemischbildung gewährleisten.

In der DE-A 4422815 wird ein Verfahren zur Herstellung von Acetylen und Synthesegas offenbart, bei welchem die Kanäle des Brennerblocks auf der Anströmseite von mit Perforationen versehenen Platten abgedeckt sind (Anspruch 1).

Die Verwirklichung dieses Konzeptes ist mit erheblichem konstruktivem Aufwand verbunden, da eine gleichmäßige Verteilung der Reaktandenströme in geeignete Mischeinheiten über alle Brennerblockbohrungen verteilt gewährleistet sein muss. Weiterhin besteht die Gefahr des Flammenrückschlagens in die einzelnen Mischrohre und der Stabilisierung der Flamme im stöchiometrischen Bereich des Mischungsfeldes. Dies gilt für den Fall einer Ungleichverteilung des Reaktionsgemisches, bei dem die Strömungsgeschwindigkeit im Mischrohr in die Größenordnung der Umsatzgeschwindigkeit sinkt.

Es stellte sich die Aufgabe, eine verbesserte Verfahren zur partiellen Oxidation von Kohlenwasserstoffen zu finden, welches die genannten Nachteile vermeidet und welches in verfahrenstechnisch einfacher Art und Weise eine schnelle und gute Vermischung der Reaktanden auf sehr kleinem Raum und bei kurzen Verweilzeiten ermöglicht.

Demgemäß wurde ein Verfahren zur partiellen Oxidation von Kohlenwasserstoffen in einem Reaktor gefunden, bei welchem man dem Reaktor einen den Kohlenwasserstoff enthaltenden Strom sowie einen den Sauerstoff enthaltenden Strom zuleitet, welches dadurch gekennzeichnet ist, dass man die beiden dem Reaktor zugeführten Ströme im Reaktor separat durch jeweils eine oder mehrere räumlich voneinander getrennte Leitungen führt, wobei diese Leitungen in ihrem Innern Turbulenzgeneratoren aufweisen, aufgrund derer durch die vorgegebene Umlenkung der Strömungsrichtung stromab dieser Turbulenzgeneratoren ein hochturbulentes Strömungsfeld ausgebildet wird und die Ströme anschließend nach Austritt aus den Leitungen in einer Mischzone vermischt und dann in einer Reaktionszone umgesetzt werden.

Bei dem erfindungsgemäßen Verfahren werden die Reaktandenströme räumlich getrennt voneinander in bevorzugt parallel verlaufende Bohrungen geführt, wobei in diesen der Strömung durch Turbulenzgeneratoren eine möglichst hohe Turbulenzstruktur aufgeprägt wird. Im Nachlauf der Turbulenzgeneratoren kommt es nun zur Gemischbildung von Oxidator und Brennstoff auf engstem Raum, wobei der Grad der Vermischung von der Mischlänge, dem Turbulenzgrad und der Drehrichtung des Turbulenzgenerators in den Bohrungen abhängt. Unmittelbar an die Mischzone schließt sich die Reaktionszone an, welche durch Eindüsen von Pilotsauerstoff in die hochturbulente Strömungszone stabilisiert wird.

Durch diese sequentielle Anordnung von Misch- und Reaktionsraum ist eine einfache Strömungsführung gewahrt, welche vorteilhafterweise nicht durch weitere Einbauten gestört wird.

Das erfindungsgemäße Verfahren bietet die Möglichkeit, durch Vorzündungen hervorgerufene Betriebsunterbrechungen und Ausfallzeiten zu unterbinden. Weiterhin eröffnen sich Möglichkeiten zum anteiligen Einsatz von Brennstoffen mit niedrigen Induktionszeiten wie Synthesegase oder höhere Kohlenwasserstoffe (beispielsweise Ethan, Ethylen oder verdampfte Flüssiggase).

Bei dem erfindungsgemäßen Verfahren werden als Turbulenzgeneratoren bezeichnete Einbauten in den Kanälen des Brennerblocks eingesetzt. Die Turbulenzgeneratoren sind in ihrer Geometrie hierbei so gestaltet, dass sie im eingesetzten Zustand wesentliche Teile des Kanalquerschnitts versperren und die Gasströmung im Innern der Kanäle nur durch in den Turbulenzgeneratoren enthaltenen, durchgängigen Bohrungen geleitet wird. Hierbei versteht man unter dem Kanalquerschnitt die dem Gas zur Durchströmung des Kanals zur Verfügung stehende Fläche.

Im Fall der meist mit einem kreisförmigen Querschnitt ausgebildeten Brennerblockkanälen weist so ein Turbulenzgenerator bevorzugt die Form eines Zylinders auf, dessen Durchmesser so bemessen ist, dass es an den Umfangsrändern dieses zylindrischen Körpers im eingebauten Zustand zu der vorstehend erläuterten Versperrung für die Gasströmung kommt, da der Außendurchmesser des Zylinders annähernd dem Innendurchmesser des Kanals entspricht und so ein Durchströmen des Gases an dem höchstens noch in sehr geringem Maße vorhandenen Spalt annähernd vollständig unterbunden wird. Die Qualität der Abdichtung wird hier insbesondere durch den Aufwand der Fertigung bestimmt und kann durch weitere, dem Fachmann bekannte Maßnahmen noch zusätzlich gesteigert werden.

Die Höhe des eingesetzten zylindrischen Körpers liegt hierbei üblicherweise in einem Bereich von 1 bis 4 Kanaldurchmessern, bevorzugt 2 bis 3 Kanaldurchmesser. Generell empfiehlt es sich dem erfindungsgemäßen Verfahren die Turbulenzgeneratoren so in ihrer Geometrie zu gestalten, dass 20 bis 100 % der Länge des Brennerkanals erfindungsgemäß im Wesentlichen versperrt sind.

Die erfindungsgemäß in den Turbulenzgenerator eingebrachten Bohrungen verlaufen im eingebauten Zustand im Wesentlichen nicht oder bevorzugt gar nicht parallel zur Längsachse des Brennerkanals. In einer bevorzugten Ausführungsform wird im Fall des vorstehend geschilderten, zylindrischen Körpers dieser mit Bohrungen mit einem Steigungswinkel von 80° bis 40°, bevorzugt 60° bis 45° versehen. Unter dem Steigungswinkel versteht man hierbei den Winkel zwischen der Längsachse des Zylinders

(Höhenachse) und der Längsachse der Bohrung. Im Fall einer axialen Ausrichtung der Bohrungen in dem Turbulenzgenerator wäre dieser Steigungswinkel 0°. Diese Bohrung wird bevorzugt an der oberen oder unteren Kreisfläche des Zylinders nahe oder am Umfangsrand angesetzt und verläuft dann mit bevorzugt konstantem Steigungswinkel spiralförmig zur gegenüberliegenden Kreisfläche. Es empfiehlt sich hierbei, den Turbulenzgenerator mit mehreren Bohrungen zu versehen, üblicherweise wählt man hier 1 bis 6, bevorzugt 4 Bohrungen.

Im eingebauten Zustand bewirken die so gestalteten Turbulenzgeneratoren, dass das Gas notwendigerweise durch die Bohrungen strömt. Hierbei strömt das Gas aufgrund des Steigungswinkels der Bohrungen in Form der vorstehend genannten, spiralförmigen Bewegung. Die Bewegungsrichtung kann sich somit zum einen weiterhin aus einer Komponente in Richtung der Längsachse zusammensetzen, andererseits ist dieser Richtung jedoch auch eine Umfangskomponente (in Abhängigkeit vom Ort der angesetzten Bohrung beispielsweise entsprechend dem Kreis gebildet durch den Durchmesser des Zylinders) überlagert. Bis das Gas den gesamten Turbulenzgenerator durchströmt hat, erfährt es hierbei eine bogenförmige Umlenkung in einem Umfangswinkel von 45° bis 360°, bevorzugt 90° bis 180°. Unter dem Umfangswinkel versteht man hierbei den überstrichenen Gesamtausschnitt des Kreisbogens, welcher durch die Zylindergeometrie gegebenen ist. Die Umlenkung kann sowohl im Uhrzeigersinn als auch gegen den Uhrzeigersinn erfolgen.

Erfindungsgemäß wird durch die Turbulenzgeneratoren ein prozentualer Flächenanteil des Kanalquerschnitts von 10% bis 70%, bevorzugt 20% bis 50% versperrt. Die äußere Geometrie der erfindungsgemäß eingesetzten Turbulenzgeneratoren wird stark durch die Geometrie des Brennerkanals vorgegeben. Im Falle kreisförmiger Kanäle empfiehlt sich wie geschildert die zylindrische Form, im Falle eines rechteckigen Querschnitts beim Brennerkanal eignet sich bevorzugt ein quaderförmiger Körper. Die jeweils besonders geeignete Ausgestaltung kann vom Fachmann ermittelt werden.

Die Durchmesser der in den Turbulenzgeneratoren anzubringenden Bohrungen liegen üblicherweise bei dem 0.1 bis 0.5, bevorzugt 0.2 bis 0.4-fachen des Zylinderdurchmessers, d.h. dem Außendurchmesser des Turbulenzgenerators.

Das Gas erfährt bei dem erfindungsgemäßen Verfahren beim Strömen durch die Bohrungen in den Turbulenzgeneratoren die vorstehend geschilderte Umlenkung. Es wird somit dem Gas ein Drall aufgegeben, es bildet sich eine verdrallte Strömung. So wird hier durch die Umlenkung des strömenden Mediums eine ausgeprägte radiale und tangentiale Geschwindigkeitskomponente am Austritt aus dem Brenner generiert. Durch die hohen Scherkräfte welche benachbart austretende Strahlen (d.h. von benachbarten Brennerkanälen) aufeinander ausüben kommt es zur Ausbildung hoher Schwankungsgeschwindigkeiten . Damit kann vorteilhafterweise in der Brennkammer die Ausbildung eines energetisch hochintensiven, turbulenten Strömungsfeldes generiert werden, in dem sich die Reaktionszone ausbilden kann. Die gebildete Turbulenz ist gekennzeichnet durch Strömungsbedingungen, bei denen hohe Schwankungsgeschwindigkeiten in allen drei Raumrichtungen ausgebildet sind und sich weiterhin keine makroskopischer Vorzug in Umfangsrichtung erkennen lässt.

Durch die erfindungsgemäße Anordnung der Turbulenzgeneratoren in den Kanälen des Brennerblocks ergibt sich eine neue und verbesserte Verfahrensführung, auf welche im Folgenden näher eingegangen wird. Erfindungsgemäß erfolgt hier eine andere Art der Stabilisierung der Reaktion. Neben der Verwendung von Halteflammen generiert der Turbulenzgenerator ein hochintensives Strömungsfeld, in dem sich die Reaktionszone ausbilden kann. Die Art des ausgebildeten Strömungsfeldes und der Verfahrensführung bewirkt vorteilhafterweise, dass hier keine Rezirkulation von Stoffströmen nahe des Brennerblocks zu beobachten ist, was letztlich dazu führt, dass hier keine Koksablagerungen am Brennerblock zu beobachten sind. Neben der bereits geschilderten vorteilhaften Ausgestaltung des Turbulenzfeldes wird die Vermeidung der unerwünschten Rezirkulation russhaltiger Gasströme an den Brennerblock auch durch die erfindungsgemäße Ausgestaltung der Positionierung der Halteflammen bewirkt. Die Halteflammen befinden sich im Gegensatz zu konventionellen Brennern in signifikantem Abstand von dem Brennerblock, bevorzugt beträgt der Abstand das 3 bis 20, besonders bevorzugt das 4 bis 15-fache besonders bevorzugt das 6-fache bis 15-fache des Durchmessers eines Kanals im Brennerblock. Dieser Abstand wird gemessen von der Unterkante des Brennerblocks, d.h. die Stelle, an welcher der Gasstrom die Kanäle in Richtung Brennraum verlässt und der Stelle, an welcher der Hilfssauerstoff in den Brennraum eingeleitet wird. Üblicherweise liegen die Durchmesser von den Kanälen in dem Brennerblock in einem Bereich von 17 bis 27 mm, bevorzugt 20 bis 23 mm. Durch diese Positionierung der Halteflammen stabilisiert sich die Flamme beim erfindungsgemäßen Verfahren deutlich weiter entfernt vom Brennerblock als bei konventionellen Brennern. Die jeweils besonders bevorzugte Positionierung der Halteflammen hängt von dem jeweiligen System und den spezifisch vorgegebenen, verfahrenstechnischen Bedingungen ab. Eine entsprechende Variation kann im Einzelfall vom Fachmann je nach den vorgegeben Reaktionsbedingungen durchgeführt werden. Eine einfache Verschiebung der Halteflammen bei konventionellen Brennern auf einen ähnlichen Abstand würde nicht vergleichbar zum Erfolg führen, da dessen Brennerbohrungen am Austritt nicht erfindungsgemäß mit Turbulenzerzeugern versehen sind und sich somit stromab des Austritts kein turbulentes Strömungsfeld einstellt, in dem die Hauptflamme nur durch Einsetzen von Hilfssauerstoff und ohne die stabilisierende Wirkung der Rezirkulationen an den Brenneraustrittsbohrungen gehalten (stabilisiert) wird.

Das erfindungsgemäße Verfahren bietet eine hohe Turbulenzerzeugung im Brennraum bei minimalem Druckverlust. Der Druckverlust ist abhängig vom Durchsatz und liegt am Auslegungspunkt des Reaktors bei 40 bis 300 mbar.

Die Turbulenzgeneratoren weisen in ihrer Anordnung bevorzugt eine alternierende Drehrichtung (im und gegen den Uhrzeigersinn) auf. Hierdurch kann man vorteilhafterweise in der Brennkammer ein turbulentes Strömungsfeld ohne eine integrale Resultierende der Tangentialgeschwindigkeit ausbilden, wodurch eine besonders effektive Durchmischung erzielt werden kann.

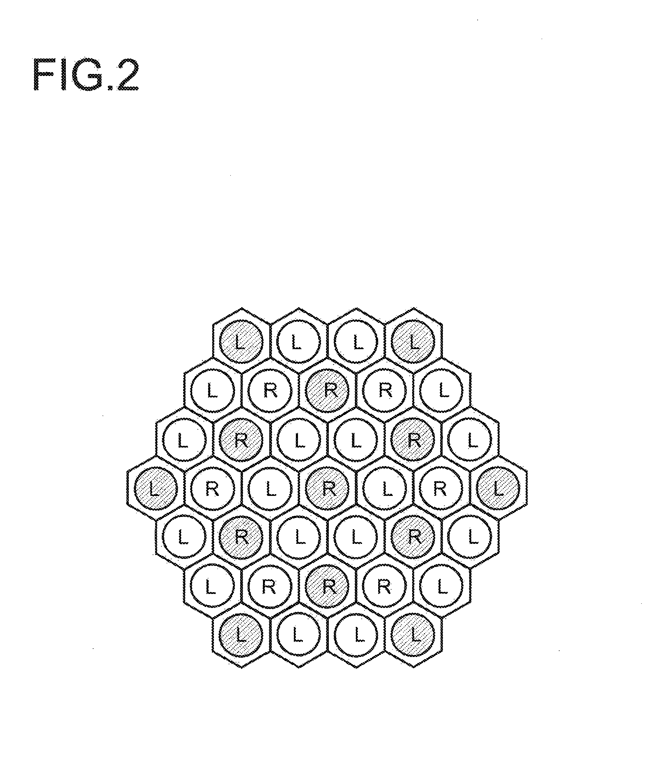

Eine bevorzugte Anordnung der Turbulenzgeneratoren sieht vor, dass in radialer Richtung die Drehrichtungen abgewechselt werden, in Umfangsrichtung dagegen Turbulenzgeneratoren gleicher Bauart eingesetzt werden. Die Anordnung von Drehrichtungen für die Oxidator und Brennstoff führenden Turbulenzerzeuger in der Brennerplatte sind in Figur 2 gezeigt.

Es können sowohl in allen Kanälen als auch nur in einigen davon Turbulenzgeneratoren angeordnet sein.

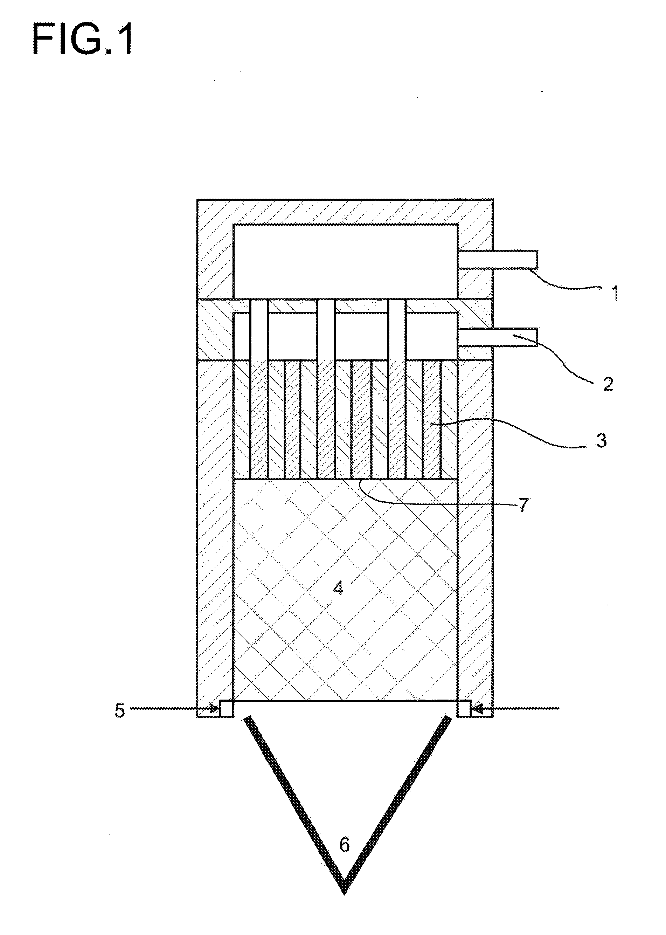

Ein weiterer Gegenstand der vorliegenden Erfindung ist eine für die Durchführung des erfindungsgemäßen Verfahrens geeignete Vorrichtung. Diese wird im Folgenden anhand der Figur 1 exemplarisch näher erläutert.

Mittel der Zuführungen (1) und (2) werden Oxidator und Brennstoff getrennt voneinander durch mit Turbulenzerzeuger versehen Leitungen (3) im Brennerblock zugeführt. Erst stromab der Turbulenzgeneratoren, die vorzugsweise aus axialen Drallerzeugern gefertigt sind, kommen Oxidator und Brennstoff in Kontakt und werden im intensiven Turbulenzfeld gemischt. Mischraum (4) ist in der Länge so bemessen, dass sie für eine ausreichende Homogenisierung der Mischung ausreicht.

Durch die Zuführungen (5) gelangt Sauerstoff oder ein Reaktionshilfsstoff in den Reaktionsraum (6), wo die Reaktionszone stabilisiert wird.

In Figur 2 ist eine bevorzugte Anordnung der Turbulenzgeneratoren dargestellt. Mit "R" und "L" sind schematisch Turbulenzgeneratoren unterschiedlicher Drehrichtung bezeichnet ("R" rechtsdrehend, "L" linksdrehend). In Umfangsrichtung finden sich demnach auf einem Kreisbogen bevorzugt Turbulenzgeneratoren gleicher Drehrichtung, während in radialer Richtung alternierende Drehrichtungen angeordnet sind. In die Leitungen, welche in der Figur 2 mit Schraffur gekennzeichnet sind, wird hierbei Oxidator eingeleitet.

Das erfindungsgemäße Verfahren ermöglicht eine wirtschaftliche partielle Oxidation von Kohlenwasserstoffen. Besonders bevorzugt eignet es sich für die Herstellung von

Acetylen und Synthesegas in hohen Ausbeuten. Im Gegensatz zu Verfahren in konventionellen Brennern eröffnet das Verfahren eine partielle Oxidation ohne eine unerwünschte Koksablagerung, welche zu einer Beeinträchtigung des Verfahrens führt. Hierbei eröffnet die erfindungsgemäße Art der Stabilisierung der Flamme im Brenner aufgrund der Vermeidung der Koksablagerung durch Rezirkulation eine effektive und wirtschaftliche Reaktionsführung. Hierbei können die Vorteile in einfacher Art und Weise durch die erfindungsgemäße strömungstechnische Ausgestaltung realisiert werden, ein erhöhter Aufwand wie beispielsweise das periodische, mechanische Abreinigen des Brenners kann so vermieden werden.

### Beispiel

Es wurden experimentelle Messungen zur Mischung in einer gemäß der Figur 1 gezeigten, erfindungsgemäßen Vorrichtung durchgeführt. Ein Reaktorraum mit einem Durchmesser von 170mm wurde mit 37 Bohrungen von 25mm versehen. Das die Bohrungen verlassende Gas wurde durch die in den Leitungen enthaltenen Turbulenzgeneratoren am Strömungsauslass der Bohrungen mit einer tangentialen Geschwindigkeitskomponente mit abwechselnder Drehrichtung bedacht.

Die Turbulenzgeneratoren zeichneten sich durch vier Bohrungen aus, welche bei einer Länge des Zylinders von 5 cm einen Winkel von 360° überstrichen.

Die Verteilung der Oxidator und Brennstoffzulässe wurde so gewählt, dass sich insgesamt eine gleichmäßig verteilte Impulsstromdichte aus den jeweiligen Bohrungen ergibt, hierzu wurde eine Anordnung, wie sie in Figur 2 dargestellt ist, ausgewählt.

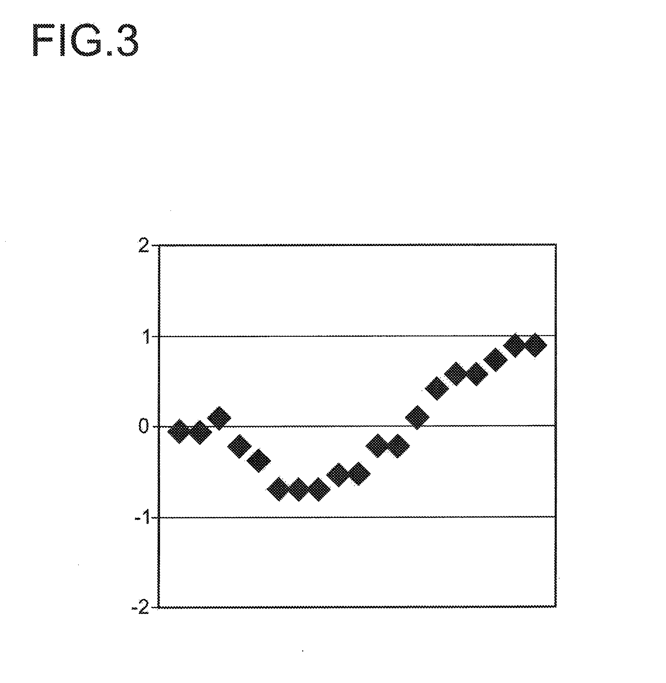

Es wurden radiale Konzentrationsmessungen zur Ermittlung der Qualität der Vermischung von Oxidator und Brennstoff auf Höhe der Flammenstabilisierung, also am Austritt der Mischstrecke und Übergang in den Reaktorraum, durchgeführt. Der Abstand zwischen Messstelle und dem Austritt der Reaktionsmedien (Bezeichnung 7 in Figur 1) betrug 8 Bohrungsdurchmesser. In dem Diagramm in Figur 3 ist die ermittelte Mischgüte über radialen Position (ermittelt wurde über den gesamten Reaktordurchmesser von 170 mm) dargestellt. Das Diagramm zeigt die prozentuale Abweichung der tatsächlich ermittelten Mischung hinsichtlich des Brennstoffgehalts (Methan) im Vergleich zu einer idealen Vermischung. Es ist festzustellen, dass die Abweichung von der idealen Vermischung bei der erfindungsgemäßen Ausgestaltung kleiner als 1 Prozent beträgt, somit eröffnet diese Ausführungsform eine effektive Verfahrensdurchführung.

## Patentansprüche

1. Verfahren zur partiellen Oxidation von Kohlenwasserstoffen in einem Reaktor, bei welchem man dem Reaktor einen den Kohlenwasserstoff enthaltenden Strom sowie einen den Sauerstoff enthaltenden Strom zuleitet, **dadurch gekennzeichnet, dass** man die beiden dem Reaktor zugeführten Ströme im Reaktor separat durch jeweils eine oder mehrere räumlich voneinander getrennte Leitungen führt, wobei diese Leitungen in ihrem Innern Turbulenzgeneratoren aufweisen, aufgrund derer durch die vorgegebene Umlenkung der Strömungsrichtung stromab dieser Turbulenzgeneratoren ein hochturbulentes Strömungsfeld ausgebildet wird, wobei die Turbolenzgeneratoren 10 bis 70% des prozentualen Flächenan- - teils des Leitungsquerschnitts versperren und die durch die Turbulenzgeneratoren geführten Ströme bei dem Durchströmen eine bogenförmige Umlenkung in einem Umfangswinkel von 45° bis 360° erfahren und die Ströme anschließend nach Austritt aus den Leitungen eine tangentiale Geschwindigkeitskomponente aufweisen und in einer Mischzone vermischt und dann in einer Reaktionszone umgesetzt werden.

2. Verfahren nach Anspruch 1, **dadurch gekennzeichnet, dass** man Acetylen und Synthesegas durch partielle thermische Oxidation herstellt.

3. Verfahren nach Ansprüchen 1 oder 2, **dadurch gekennzeichnet, dass** man in sämtlichen Leitungen Turbulenzgeneratoren einsetzt.

4. Verfahren nach Ansprüchen 1 bis 3, **dadurch gekennzeichnet, dass** man die Turbulenzgeneratoren in den Leitungen so anordnet, dass sich bei dem Durchströmen des Stoffstroms durch die Kanäle bezogen auf benachbarte Kanäle eine alternierende Drehrichtung ausbildet.

5. Verfahren nach Ansprüchen 1 bis 4, **dadurch gekennzeichnet, dass** man zur Ausbildung einer geeigneten Mischung der Reaktionspartner die zur Flammenstabilisierung verwendete Halteflamme in einem Abstand von dem 6-fachen bis 15-fachen des Kanaldurchmessers von der Unterkante des Brennerblocks gemessen im Brennraum anordnet.

6. Vorrichtung zur Durchführung der Verfahren gemäß Ansprüchen 1 bis 5, welche einen Reaktor zur Acetylenherstellung umfasst, wobei in dem Reaktor bezogen auf die Strömungsrichtung der Einsatzstoffe vor der Mischzone der Einsatzstoffe separate Leitungen enthalten sind, in welchen räumlich getrennt der den Kohlenwasserstoff enthaltende Strom bzw. der den Sauerstoff enthaltende Strom geführt werden, wobei im Innern einer oder mehrerer dieser Leitungen Turbulenzgeneratoren enthalten sind, wobei die Turbolenzgeneratoren 10 bis 70% des prozentualen Flächenanteils des Leitungsquerschnitts versperren und die durch die Turbulenzgeneratoren geführten Ströme bei dem Durchströmen eine bogenförmige Umlenkung in einem Umfangswinkel von 45° bis 360° erfahren und diese Leitungen mit ihren Enden in die Mischzone münden, in welcher die zugegebenen Einsatzstoffe vermischt und zur Reaktion gebracht werden.

7. Vorrichtung nach Anspruch 6, wobei die in der oder den Leitungen angeordneten Turbulenzgeneratoren den Leitungsquerschnitt zu 10 bis 70% versperren.

8. Vorrichtung nach Anspruch 6 oder 7, wobei die Turbulenzgeneratoren Bohrungen für die Durchleitung eines Stoffstromes aufweisen und diese Bohrungen mit einem Steigungswinkel von 40 bis 80° bezogen auf die Längsachse der Leitung angeordnet sind.

## Claims

1. A process for partial oxidation of hydrocarbons in a reactor, in which a stream comprising the hydrocarbon and a stream comprising the oxygen are fed to the reactor, wherein both streams fed to the reactor are conducted within the reactor separately through in each case one or more spatially separate lines, these lines having turbulence generators in their interior, owing to which, as a result of the imposed deflection of the flow direction downstream of said turbulence generators, a highly turbulent flow field forms, the turbulence generators blocking 10 to 70% of the percentage area of the flow cross section and the streams conducted through the turbulence generators, as they flow through, undergoing curved deflection in a circumference angle of from 45° to 360°, and the streams then having a tangential flow component after exiting from the lines and being mixed in a mixing zone and then converted in a reaction zone.

2. The process according to claim 1, wherein acetylene and synthesis gas are prepared by partial thermal oxidation.

3. The process according to claim 1 or 2, wherein turbulence generators are used in all lines.

4. The process according to claims 1 to 3, wherein the turbulence generators are disposed in the lines such that, as the stream flows through the channels, based on adjacent channels, an alternating direction of rotation is formed.

5. The process according to claims 1 to 4, wherein a suitable mixture of reactants is formed by disposing the stabilizing flame at a distance of from 6 times to 15 times the channel diameter from the lower edge of the burner block measured in the burner space.

6. An apparatus for performing the process according to claims 1 to 5 which comprises a reactor for acetylene preparation, said reactor comprising, based on the flow direction of the feed stocks upstream of the mixing zone of the feed stocks, separate lines in which, spatially separately, the stream comprising the hydrocarbon and the stream comprising the oxygen are conducted, the interior of one or more of these lines comprising turbulence generators, the turbulence generators blocking 10 to 70% of the percentage area of the flow cross section and the streams conducted through the turbulence generators, as they flow through, undergoing curved deflection in a circumference angle of from 45° to 360°, and the ends of these lines opening into the mixing zone in which the feed stocks added are mixed and reacted.

7. The apparatus according to claim 6, wherein the turbulence generators arranged in the line(s) block the line cross section to an extent of from 10% to 70%.

8. The apparatus according to claim 6 or 7, wherein the turbulence generator(s) has/have bores for the passage of a stream and these bores are arranged with a slope of from 40° to 80° based on the longitudinal axis of the line.

## Revendications

1. Procédé pour l'oxydation partielle d'hydrocarbures dans un réacteur, dans lequel on envoie au réacteur un courant contenant l'hydrocarbure ainsi qu'un courant contenant l'oxygène, **caractérisé en ce qu'**on fait passer séparément dans le réacteur les deux courants envoyés au réacteur chacun par un ou plusieurs conduits séparés les uns des autres dans l'espace, ces conduits comportant dans leur intérieur des générateurs de turbulence grâce auxquels un champ d'écoulement hautement turbulent est formé en aval de ces générateurs de turbulence par la déviation préétablie du sens d'écoulement, les générateurs de turbulence bloquant 10 à 70 % de la quantité de surface en pourcentage de la section transversale des conduits et les courants envoyés à travers les générateurs de turbulence subissant lors du passage une déviation en forme d'arc sous un angle inscrit de 45° à 360° et les courants présentant ensuite après sortie des conduits une composante de vitesse tangentielle et étant mélangés dans une zone de mélange et ensuite mis en réaction dans une zone de réaction.

2. Procédé selon la revendication 1, **caractérisé en ce qu'**on produit de l'acétylène et du gaz de synthèse par oxydation thermique partielle.

3. Procédé selon la revendication 1 ou 2, **caractérisé en ce qu'**on utilise des générateurs de turbulence dans la totalité des conduits.

4. Procédé selon les revendications 1 à 3, **caractérisé en ce qu'**on dispose les générateurs de turbulence dans les conduits de manière qu'un sens de rotation alterné apparaisse lors du passage du courant de substances dans les canaux, par rapport aux canaux voisins.

5. Procédé selon les revendications 1 à 4, **caractérisé en ce que** pour la formation d'un mélange convenable des partenaires réactionnels on dispose la flamme d'entretien, utilisée pour la stabilisation de la flamme, à une distance représentant 6 fois à 15 fois le diamètre du canal, mesurée à partir du bord inférieur du bloc brûleur.

6. Dispositif pour la mise en oeuvre du procédé selon les revendications 1 à 5, qui comprend un réacteur pour la production d'acétylène, dans le réacteur étant contenus des conduits séparés par rapport au sens d'écoulement des produits de départ avant la zone de mélange des produits de départ, dans lequel le courant contenant l'hydrocarbure et respectivement le courant contenant l'oxygène sont envoyés en étant séparés dans l'espace, à l'intérieur d'un ou de plusieurs de ces conduits étant contenus des générateurs de turbulence, les générateurs de turbulence bloquant 10 à 70 % de la quantité de surface en pourcentage de la section transversale des conduits et les courants envoyés à travers les générateurs de turbulence subissant lors du passage une déviation en forme d'arc sous un angle inscrit de 45° à 360° et ces conduits débouchant par leur extrémité dans la zone de mélange, dans laquelle les produits de départ ajoutés sont mélangés et mis en réaction.

7. Dispositif selon la revendication 6, dans lequel les générateurs de turbulence disposés dans le ou les conduits bloquent à raison de 10 à 70 % la section transversale des conduits.

8. Dispositif selon la revendication 6 ou 7, dans lequel les générateurs de turbulence présentent des trous pour le passage d'un courant de substance et ces trous sont disposés en faisant un angle d'inclinaison de 40 à 80° par rapport à l'axe longitudinal du conduit.
